# EUROPEAN PATENT SPECIFICATION

(11) **EP 4 040 951 B1**
(45) Date of publication and mention of the grant of the patent: **19.07.2023**
(21) Application number: 20785749.1
(22) Date of filing: 05.10.2020
(51) Int. Cl.: A01K 29/00

(54) **A CONTROL SYSTEM FOR ADAPTING A LIGHT RECIPE**
STEUERUNGSSYSTEM ZUR ANPASSUNG EINES LICHTREZEPTS
SYSTÈME DE COMMANDE POUR ADAPTER UNE RECETTE DE LUMIÈRE

(30) Priority: 10.10.2019 EP 19202525
(43) Date of publication of application: 17.08.2022
(73) Proprietor: Signify Holding B.V., 5656 AE Eindhoven (NL)
(72) Inventor: DE SAMBER, Marc Andre, 5656 AE Eindhoven (NL); BROERS, Harry, 5656 AE Eindhoven (NL)
(74) Representative: Verweij, Petronella Daniëlle
(86) International application number: PCT/EP2020/077848
(87) International publication number: WO 2021/069375

(56) References cited:
- WO-A1-2017/159491
- CN-A- 107 242 152
- US-A- 4 625 728
- US-B2- 10 237 956

## Description

### FIELD OF THE INVENTION

The invFention relates to a control system for adapting a light recipe in a growing cycle of at least one boar, so as to delay sexual maturation of said at least one boar. The invention further relates to a method of adapting a light recipe in a growing cycle of at least one boar; and to a corresponding computer program product.

### BACKGROUND OF THE INVENTION

Different mammalian species show a wide diversity in methods of breeding. Some mammals limit their reproductive activity to a certain period of the year, while others breed throughout the year. Breeding and breeding periods during the lifecycle of a mammalian animal may have consequences on meat quality for human consumption. Namely: Boar taint is a serious problem in the breeding of boars for the production of meat. A boar is a (uncastrated) male pig, or uncastrated male swine. Boar taint is an unpleasant odor that emanates from boar fat (of the boar meat) when heated, e.g. during cooking. Boar taint develops when a boar reaches puberty and sexually matures. Commonly, up to a fifth of produced pork meat copes with the undesired consequences of boar taint.

Castration of male piglets may be a solution to prevent said male piglets coming into puberty and get sexually mature, and hence develop boar taint. However, castration is causing more and more concerns in terms of animal welfare. Moreover, the physical castration leads to a reduced weight-to-feed efficiency and may lead to a higher fat content in the pig. Administration of exogenous chemical castration substances, which may be an alternative to physical castration, is also not wanted by consumers. Thus, it may be desired to reduce and/or prevent boar taint, while maintaining animal welfare.

CN107242152A discloses a control system for adapting a light recipe in a growing cycle of a boar. WO2017159491A1 discloses a method of raising livestock based on circadian cycles.

### SUMMARY OF THE INVENTION

It is found that a light recipe may influence breeding (and/or pre-breeding) physiology of at least one boar. The growing cycle of at least one boar may therefore be accompanied by a light recipe providing artificial lighting conditions (i.e. e.g. in stables). Such artificial lighting conditions may for example be the mimicking of seasonal conditions, such as providing variations in the length and/or intensity of daylight, or the application of circadian rhythms. Hence, such a light recipe may be characterized by a particular spectrum, a photo-period and/or a light intensity. These parameters may namely be set (dynamically) to create a 'baseline' for growing the at least one boar with said light recipe. The at least one boar may thereby be illuminated with said light recipe during its growing cycle or its lifecycle.

Moreover, boars may breed year-round in artificial lighting conditions, and are typically seasonal short-day breeders. Hence, under lighting conditions mimicking end-of-summer and/or autumn, the at least one boar will gradually sexually mature and become ready for mating and/or breeding. In contrast, under lighting conditions mimicking summer (i.e. e.g. long periods of bright light), the at least one boar is found not to hormonally develop towards adultery and shows immature spermatogenesis. Endogenous melatonin production may be one of the regulators for this development. Signify has significant experience in regulating melatonin production with lighting conditions.

Considering the above, it is an object of the invention to provide an improved control system for adapting a light recipe in a growing cycle of at least one boar, so as to delay sexual maturation of said at least one boar, which at least alleviates the problems and disadvantages related to boar taint mentioned above. Thereto, the invention provides a control system for adapting a light recipe in a growing cycle of at least one boar to delay sexual maturation of said at least one boar, wherein the control system comprises a controller configured to: control a lighting device to illuminate the at least one boar with said light recipe; obtain a first signal indicative of a sexual maturation level of the at least one boar; control, upon determining that the sexual maturity level of the at least one boar exceeds a predefined sexual maturity level, the lighting device to adapt said light recipe by (i) increasing a blue content of a spectrum of said light recipe, (ii) increasing a length of a photo-period of said light recipe, and/or (iii) increasing a light intensity of said light recipe. Here, the definition of a boar may mutatis mutandis be phrased as a swine, or a male swine, throughout.

The control system according to the invention comprises a controller configured to control a lighting device to illuminate the at least one boar with a light recipe, such as partly mentioned above. The controller of the control system is further configured to obtain a first signal indicative of the sexual maturation level of the at least one boar. Upon determining that the sexual maturity level of the at least one boar exceeds a predefined sexual maturity level, the controller controls the lighting device to adapt said light recipe. Thereby, said predefined sexual maturity level may be the onset of puberty of a boar, which is a stage of sexual maturity after which boar taint increasingly manifests itself (as mentioned). Said adapting of the light recipe comprises (i) increasing a blue content of a spectrum of said light recipe, (ii) increasing a length of a photo-period of said light recipe, and/or (iii) increasing a light intensity of said light recipe.

More specifically: By increasing the blue content of the spectrum of said light recipe, the melatonin generation may be regulated without changing the overall perception of the color of the light as e.g. white. Moreover, by increasing the photo-period and/or said light intensity, the lighting characteristics of summer may increasingly be mimicked in a later stage of the growth cycle of the at least one boar.

The increase of the values of these parameters maintains the implemented light recipe, but delays the sexual maturation of said at least one boar, because the adapted conditions mimic less favorable breeding conditions within the growth cycle of the at least one boar. Therefore, as a result of the control system according to the invention, boar taint may advantageously be reduced and/or prevented by adapting the light recipe in the growing cycle of the at least one boar, because the at least one boar is delayed in sexual maturation and will be less sexually mature upon slaughter.

The first signal may be a wireless signal. In an embodiment, the control system may comprise the lighting device. The lighting device may for example be a luminaire. The lighting device may be a spotlight or illuminating tile. The lighting device may be installed in a stable. The at least one boar may be one boar, or a population of boars, or phrased as a population of pigs. The controller according to the invention may have processing power and internal memory capabilities to perform the functions according to the invention. The controller may be equipped with local or cloud-accessible image analysis tools, which may be complemented with artificial intelligence and/or machine learning.

The control system according to the invention may similarly be a lighting control system, or an illumination system. The illumination system may be a connected and tunable illumination system. The illumination system may be installed in a livestock stable, pen, or processing station; thereby allowing real-time and timed control of the light along a range of lighting parameters such as on/off, dimming, color tuning, spectral tuning, sequencing of (mentioned) lighting parameters (e.g. photo-periods)

In an embodiment, the first signal may be indicative of the sexual maturation level of the at least one boar exceeding the predefined sexual maturation level. Hence, the first signal may originate from a processing device already determining that the sexual maturation level of the at least one boar exceeds the predefined sexual maturation level. In aspects, the control system may comprise said processing device. Said processing device may for example be part of a sensing device, such as a camera, temperature sensor, scent sensor, Time-of-Flight sensor, microphone, or a combination thereof (e.g. in a sensor bundle). The processing device may alternatively be an analysis device for analyzing ambient air, blood, animal feces, animal urine, animal hormones and/or pheromones, etc. The control device may in examples also comprise said analysis device. Said processing device may for example be an inspection tool, or user input device, such as for example of a veterinarian. The processing tool may comprise testing blood, as mentioned.

Alternatively, in an embodiment, the controller may be configured to: determine the sexual maturation level of the at least one boar based on the first signal; and determine that the sexual maturity level of the at least one boar exceeds a predefined sexual maturity level. Hence, the first signal may be indicative of the sexual maturation level of the at least one boar, and the controller itself may determine the sexual maturation level based on said first signal. The first signal may for example comprise vision data or acoustic data. Subsequently, either the controller may determine itself that the sexual maturity level of the at least one boar exceeds a predefined sexual maturity level, e.g. by comparing said sexual maturity level with a prestored predefined sexual maturity level; or the controller may determine that the sexual maturity level of the at least one boar exceeds the predefined sexual maturity level by e.g. an external processing unit performing this determination for the controller and being in communication with the controller.

In a further embodiment, the first signal may be indicative of at least one feature characterizing the sexual maturation level of the at least one boar. Moreover, the controller may be configured to determine the sexual maturation level of the at least one boar by counting a number of occurrences of said at least one feature, and/or by assessing an intensity of said at least one feature.

The at least one feature characterizing the sexual maturation level of the at least one boar may for example be a behavioral feature or a physiological feature. Hence, in a further embodiment, the at least one feature may comprise at least one behavioral feature and/or at least one physiological feature of the at least one boar. Alternatively, the at least one feature may comprise at least one physical feature. Alternatively, the at least one feature may comprise at least one biochemical feature. In an embodiment, said at least one behavioral feature may comprise an occurrence of the at least one boar: fighting another animal, jumping onto another animal, biting, being agitated, and/or performing a sexual activity. It is namely known that the onset of sexual maturation (i.e. the boar entering puberty) leads to social behavioral change, such as aggressive and excessive sexual behavior. Moreover, in an embodiment, said at least one physiological feature of the at least one boar may comprise: a musculature, a testicular size, a body smell, a skin status, a stress level, a perspiration rate, a hormone level, a pheromone level, and/or a core temperature. For example, the at least one feature may be testicular growth of the at least one boar, etc.

In a further embodiment, the control system may comprise a sensing device for determining said at least one feature; and wherein the controller may be configured to retrieve or obtain said first signal from the sensing device. Thus, the sensing device may determine said at least one feature, and convey the first signal to the controller.

Based on the at least one feature, the sexual maturation level may be determined. The sensing device does not necessarily have to be coupled to the lighting device and/or lighting system. The sensing device may be a distributed sensing device, e.g. part of a sensor network or standalone sensor, or wearable sensor. The sensing device may be in communication with or be even part of the control system according to the invention.

In a further embodiment, the sensing device may be a camera for obtaining vision data and/or a microphone for measuring acoustic data; wherein the first signal may comprise respectively the vision data and/or the acoustic data.

The sensing device may alternatively be a temperature sensor, or a scent sensor. The sensing device may alternatively be a thermal camera for obtaining temperature data; a laser scanner, Time-of-Flight sensor, or a 3D camera for measuring body shape and/or weight (e.g. measuring testicular growth). The sensing device may alternatively be a spectrum analyzer for chemical decomposition (for example, when boars become sexually aroused, they salivate profusely dispersing pheromones into the air). The sensing device may alternatively be a weighting scale. The sensing device may be a combination of the above sensors and/or alternatives, e.g. in a sensor bundle.

For example: The sexual maturity level of the at least one boar may be a function of the number of jumps of the at least one boar onto another swine. The first signal may comprise vision data indicative of the at least one feature characterizing the sexual maturation level of the at least one boar, wherein the at least one feature is the boar jumping onto another swine. The controller may subsequently determine by means of image analysis that the sexual maturity level of the at least one boar is at a particular level by counting the number of jumps of the at least one boar onto another swine. If the determined sexual maturity level of the at least one boar exceeds a predefined sexual maturity level, the controller may control the lighting device to adapt said light recipe as discussed. A similar example applies to the first signal comprising acoustic data, i.e. e.g. the sexual maturity level of the at least one boar may be a function of fighting behavior (activity) of the at least one boar, which may be measured by a microphone. Fighting behavior and increased activity of the at least one boar may also be determined by a thermal camera evaluating the body temperature of the at least one boar. Similar examples may be envisioned based on the features and sensors mentioned before.

As partly mentioned, boar taint increasingly manifests itself in meat of a boar that is slaughtered when the boar has reached puberty and has sexually matured. A boar is harvested when mass of the boar is economically sufficient. Mass of a boar increases in time during the growth cycle of the boar, but in time, the boar may also undesirably sexually mature. Hence, it may be desired to harvest a boar with an economically attractive weight, but prevent and/or delay such boar to sexually mature.

In an embodiment, the controller may be configured to output, upon determining that the sexual maturity level of the at least one boar exceeds a predefined sexual maturity level, a harvesting signal for harvesting the at least one boar. The harvesting signal may for example be a wireless signal. The harvesting signal may be a notification (signal) to harvest the at least one boar. The notification (signal) may for example be conveyed to a user device. In such an embodiment, the at least one boar may advantageously be harvested when the at least one boar reaches the stage of sexual maturity corresponding to the predefined sexual maturity level, i.e. e.g. before reaching puberty so as to reduce and/or prevent boar taint.

A harvesting signal for harvesting the at least one boar, or a boar of the at least one boar, may be advantageous, because a boar has a faster growth curve than a female pig. Hence, considering mixed-sex stables, by harvesting the boar at the desired weight, and not the female pig, the female pig may still be able to grow.

Alternatively, in an embodiment, the controller may be configured to: obtain a second signal indicative of an average weight of the at least one boar; output, upon determining a second condition in which the average weight of the at least one boar exceeds a predefined average weight of the at least one boar, a harvesting signal for harvesting the at least one boar. The harvesting signal may for example be a wireless signal. The harvesting signal may be a notification (signal) to harvest the at least one boar. The notification (signal) may for example be conveyed to a user device. Hence, the control system advantageously delays sexual maturation of the at least one boar, but outputs a harvesting signal upon determining that also the average weight of the at least one boar is sufficient for harvest, i.e. at least exceeding the predefined average weight of the at least one boar. Said average weight for harvest may be found in literature. Therefore, at least one boar may still increase in weight and may still be harvested at a desired weight, while the control system according to the invention has delayed the sexual maturity of said at least one boar. The controller may for example determine said predefined average weight from a prestored growth curve of the boar.

Thereby, in an embodiment, the second signal may be indicative of the average weight of the at least one boar exceeding a predefined average weight of the at least one boar. Moreover, in examples the second signal may be indicative of the average weight of a boar of the at least one boar exceeding a predefined average weight. Hence, the second signal may originate from a processing device already determining that the average weight of the at least one boar exceeds the predefined average weight. In aspects, the control system may comprise said processing device. Said processing device may for example be part of a sensing device, such as a weight sensor.

Alternatively, in an embodiment, the controller may be configured to: determine the average weight of the at least one boar based on the second signal; and determine that the average weight of the at least one boar exceeds a predefined weight of the at least one boar. Hence, the second signal may be indicative of an average weight of the at least one boar, and the controller itself may determine the average weight based on said second signal. The second signal may for example comprise vision data. Subsequently, the controller may determine itself that the average weight of the at least one boar exceeds a predefined average weight by performing image analysis to find the average weight and compare this to said predefined average weight. Moreover, in examples the controller may be configured to: determine the average weight of a boar of the at least one boar based on the second signal; and determine that the average weight of the boar of the at least one boar exceeds a predefined weight.

The harvesting signal may be a control signal arranged for controlling a lighting device to illuminate a location of the at least one boar. The harvesting signal may be a control signal arranged for indicating a location of the at least one boar, e.g. so as to direct harvesting to the location of the at least one boar. The harvesting signal may be an identification signal of the at least one boar, such as an identification signal pointing to a tag of the at least one boar, such as a RFID, NFC, or QR tag. Hence, in an embodiment, the harvesting signal may be a control signal configured to control the lighting device according to the invention.

In aspects of the invention: the controller may be configured to obtain an identity and/or a location of the at least one boar. The harvesting signal may comprise said identity and/or location of the at least one boar. The first signal may comprise data indicative of said identity and/or location of the at least one boar, wherein the controller may determine said identity and/or location of the at least one boar based on said data.

It is further an object of the invention to provide an improved method of adapting a light recipe in a growing cycle of at least one boar, so as to delay sexual maturation of said at least one boar, which at least alleviates the problems and disadvantages related to boar taint mentioned above. Thereto, the invention provides a method of adapting a light recipe in a growing cycle of at least one boar to delay sexual maturation of said at least one boar, wherein the method comprises: controlling a lighting device to illuminate the at least one boar with said light recipe; obtaining a first signal indicative of a sexual maturation level of the at least one boar; controlling, upon determining that sexual maturity level of the at least one boar exceeds a predefined sexual maturity level, the lighting device to adapt said light recipe by (i) increasing a blue content of a spectrum of said light recipe, (ii) increasing a length of a photo-period of said light recipe, and/or (iii) increasing a light intensity of said light recipe. Thereby, advantages and/or embodiments applying to the control system according to the invention may mutatis mutandis apply to said method according to the invention.

In an embodiment, the first signal is indicative of the sexual maturation level of the at least one boar exceeding the predefined sexual maturation level. In an embodiment, the method comprises: determining the sexual maturation level of the at least one boar based on the first signal.

In an embodiment, the first signal is indicative of at least one feature characterizing the sexual maturation level of the at least one boar, wherein the method comprises: determining the sexual maturation level of the at least one boar by counting a number of occurrences of said at least one feature, and/or by assessing an intensity of said at least one feature.

In an embodiment, the method comprises: obtaining a second signal indicative of an average weight of the at least one boar; outputting, upon determining that the average weight of the at least one boar exceeds a predefined average weight of the at least one boar, a harvesting signal for harvesting the at least one boar.

The invention further relates to a computer program product. Hence, the invention provides a computer program product for a computing device, the computer program product comprising computer program code to perform a method according to the invention when the computer program product is run on a processing unit of the computing device.

Thus, aspects of the invention may be implemented in a computer program product, which may be a collection of computer program instructions stored on a computer readable storage device which may be executed by a computer. The instructions of the present invention may be in any interpretable or executable code mechanism, including but not limited to scripts, interpretable programs, dynamic link libraries (DLLs) or Java classes. The instructions can be provided as complete executable programs, partial executable programs, as modifications to existing programs (e.g. updates) or extensions for existing programs (e.g. plugins). Moreover, parts of the processing of the present invention may be distributed over multiple computers or processors.

Moreover, in aspects, phrased differently, the controller may create an adapted light scene and control the lighting device to illuminate the at least one boar with said adapted light scene, upon determining that the sexual maturity level of the at least one boar exceeds a predefined sexual maturity level; wherein the adapted light scene is created by increasing a blue content of a spectrum of said light recipe, (ii) increasing a length of a photo-period of said light recipe, and/or (iii) increasing a light intensity of said light recipe.

In aspects, the present invention may mutatis mutandis be applied to other livestock animals, such as other mammalian species or fowl species, or applied to other aquatic animals. Hence, the adaptation of a lighting recipe may be based on measurements of relevant physiological and non-physiological parameters of said other livestock animals. This may improve productivity and meat quality and wellbeing of such livestock animals or aquatic animals. Alternatively, said livestock animal may be changed with insect animals.

Hence, in aspects, the invention may provide: a control system for adapting a light recipe in a growing cycle of at least one livestock animal to delay sexual maturation of said at least one livestock animal, wherein the control system comprises a controller configured to: control a lighting device to illuminate the at least livestock animal with said light recipe; obtain a first signal indicative of a sexual maturation level of the at least one livestock animal; control, upon determining that the sexual maturity level of the at least one livestock animal exceeds a predefined sexual maturity level, the lighting device to adapt said light recipe by (i) increasing a blue content of a spectrum of said light recipe, (ii) increasing a length of a photo-period of said light recipe, and/or (iii) increasing a light intensity of said light recipe. Thereby, advantages and/or embodiments applying to the control system and method according to the invention may mutatis mutandis apply to this aspect according to the invention.

Hence, in aspects, the invention may provide: a control system for adapting a light recipe in a growing cycle of at least one aquatic animal to delay sexual maturation of said at least one aquatic animal, wherein the control system comprises a controller configured to: control a lighting device to illuminate the at least aquatic animal with said light recipe; obtain a first signal indicative of a sexual maturation level of the at least one aquatic animal; control, upon determining that the sexual maturity level of the at least one aquatic animal exceeds a predefined sexual maturity level, the lighting device to adapt said light recipe by (i) increasing a blue content of a spectrum of said light recipe, (ii) increasing a length of a photo-period of said light recipe, and/or (iii) increasing a light intensity of said light recipe. The aquatic animal may be fish. Thereby, advantages and/or embodiments applying to the control system and method according to the invention may mutatis mutandis apply to this aspect according to the invention.

In aspects, throughout the application, the phrasing of 'the at least one boar' may be `a boar of the at least one boar' .

### BRIEF DESCRIPTION OF THE DRAWINGS

The invention will now be further elucidated by means of the schematic non-limiting drawings:
Fig. 1 depicts schematically a control system according to the invention;
Fig. 2 depicts schematically a control system according to the invention;
Fig. 3 depicts schematically a control system according to the invention;
Fig. 4 depicts schematically an embodiment of a method according to the invention.

### DETAILED DESCRIPTION OF THE EMBODIMENTS

As mentioned before, the meat processing industry copes with problems associated with boar taint, and one of the solutions to mitigate said problems is castration of piglets. However, physical castration is both disadvantageous to the animal and negatively affects animal welfare, as well as disadvantageous to the weight-to-feed ratio of the livestock. Chemical castration is undesired from a consumer viewpoint and associated consumer welfare. Hence, it may be desired to reduce and/or prevent boar taint, while maintaining e.g. animal welfare.

As partly mentioned, a growing cycle of at least one boar may be accompanied by a light recipe providing artificial lighting conditions. Such artificial lighting conditions may for example be the mimicking of seasonal conditions, such as providing variations in the length and/or intensity of daylight, or the application of circadian rhythms. Boars may therefore breed year-round in artificial lighting conditions, and are typically seasonal short-day breeders. Hence, under lighting conditions mimicking end-of-summer and/or autumn, the at least one boar will gradually sexually mature and become ready for mating and/or breeding. If the at least one boar is slaughtered after reaching puberty and sexual maturity, boar taint will increasingly manifest itself.

The control system and methods according to the present invention advantageously adapt various parameters of a light recipe in a growing cycle of at least one boar to delay sexual maturation of said at least one boar, upon determining that the sexual maturation level of at least one boar exceeds a predefined sexual maturation level; which results in a reduction and/or prevention of boar taint (of the meat of such at least one boar).

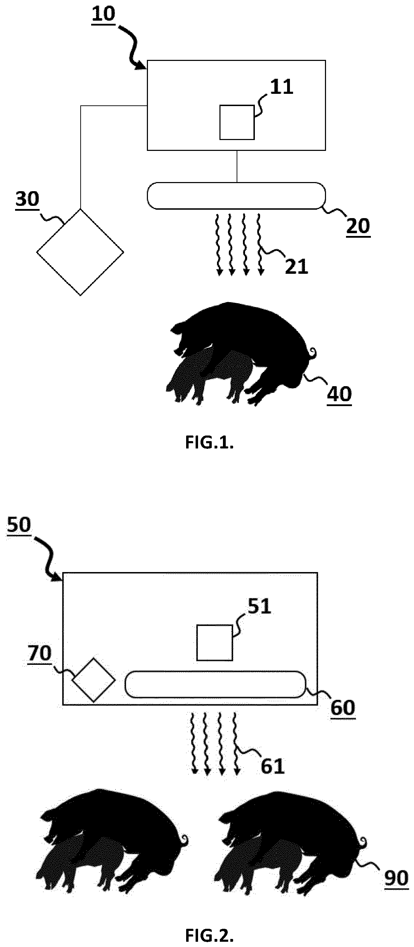

Figure 1 depicts schematically, by non-limiting example, a control system 10 according to the invention. The control system 10 is arranged for adapting a light recipe 21 in a growing cycle of two boars 40, so as to delay sexual maturation of said two boars 40. Alternatively, said two boars may be one boar, at least one boar, such as a population of boars, or a population of mixed-sex pigs comprising boars. The two boars 40 are in a stable. The light recipe 21 is thus associated with the growing cycle of the two boars 40. The light recipe 21 is initially characterized by a spectrum, a photoperiod, and a light intensity. A lighting device 20 illuminates the two boars 40 with the light recipe 21 accordingly. The lighting device 20 is a stable luminaire.

The control system 10 comprises a controller 11. The controller 11 controls the lighting device 20 to illuminate the two boars 40 with the light recipe 21. The controller 11 is in wired connection with the lighting device 20, but may alternatively be at e.g. a remote location and e.g. in wireless connection with the lighting device, via e.g. the wireless modalities such as Bluetooth, ZigBee, Wi-Fi, VLC, RF, IR, etc.

The controller 11 further obtains a first signal. The first signal is indicative of a sexual maturation level of the two boars 40. More specifically, here, the first signal is indicative of the sexual maturation level of the two boars exceeding a predefined sexual maturity level. The predefined sexual maturity level is the onset of puberty of a boar. The first signal is namely (already) determined by the processing device 30. The first signal may alternatively be indicative of the sexual maturation level of one (individual) boar of the two boars. The invention may apply mutatis mutandis for such individual boar.

The processing device 30 comprises an image sensor and a processor equipped with image analysis capabilities (not necessarily in the same housing). The image sensor records visual data associated with the two boars 40 and the corresponding processor analyses the behavior of the two boars 40. The behavioral features of the two boars 40, such as the number and/or intensity of occurrences of the two boars 40 fighting each other, of jumping onto each other, of biting, and/or of performing sexual activities may be evaluated; and as a function thereof a sexual maturity level may be determined. Hence, the processing device 30 is able to provide said first signal indicative of the sexual maturation level of the two boars exceeding a predefined sexual maturity level by means of image analysis. Alternatively, said determination may be performed per individual boar. Alternatively, said vision sensor may be any other sensing means for determining sexual maturity levels of the two boars, such as a microphone measuring acoustic data indicative of features of aggression and/or fighting boars (which may be an indication of behavior belonging to an onset of puberty of the boars). Yet alternatively, said processing device may be an analysis device for analyzing physical or physiological properties of the two boars, or properties of the stable, so as to arrive at the sexual maturity levels of the two boars. Yet alternatively, said first signal may be provided by a user device receiving a user input indicative that the sexual maturity level of the boars exceeds a predefined sexual maturity level.

Still referring to figure 1, the control system 10 obtains the first signal from the processing device 30 via a wired connection. Alternatively, said processing device may convey the first signal to the control system 10 via at least a wireless connection, such as e.g. Bluetooth, ZigBee, Wi-Fi, Lo-Ra, VLC, RF, IR, etc. The first signal indicates that the sexual maturation level of the two boars 40 exceeds the predefined sexual maturity level. Alternatively, it may be detected that the sexual maturation level of one of the two boars exceeds the predefined sexual maturity level. The controller 11 determines thereby that the sexual maturity level of the two boars 40 exceeds the predefined sexual maturity level by obtaining the first signal. Subsequently, upon determining that the sexual maturity level of the two boars 40 exceeds the predefined sexual maturity level, the controller 11 controls the lighting device 20 to adapt said light recipe 21. Here, the length of the photo-period of said light recipe is increased, and the light intensity of said light recipe 21 is increased. Thus, for example, the daily dose of artificial lighting is having a longer daily duration (i.e. e.g. summer days are longer) and is having a higher intensity (i.e. e.g. mimicking more sunny conditions). This matches for example summer conditions, and differentiates from autumn conditions, thereby delaying the sexual maturation of the two boars 40 (since boars are short-day breeders). Alternatively, the controller may control the lighting device to increase the blue content of the spectrum of the light recipe 21.

As a result, the sexual maturity of the two boars 40 is delayed, because parameters of the light recipe 21 are adapted accordingly as discussed. Boar taint may therefore be reduced and/or prevented, because the two boars 40 will be less sexually mature upon slaughter.

Figure 2 depicts schematically, by non-limiting example, a control system 50 according to the invention. The control system 50 is arranged for adapting a light recipe 61 in a growing cycle of a population of mixed-sex swine 90, the population of mixed-sex swine comprising at least one boar, so as to delay sexual maturation of said at least one boar. The light recipe 21 is initially characterized by a spectrum, a photoperiod, and a light intensity. The control system 50 comprises a controller 51, a lighting device 60 and a sensor bundle 70. The controller 51, lighting device 60 and the sensor bundle 70 are embodied within a single housing, but may alternatively be part of a distributed connected system. The controller 51 controls the lighting device 60 to illuminate the population of mixed-sex swine 90 with the light recipe 61. The light recipe 61 is tailored to the growing cycle of the population of mixed-sex swine 90.

The sensor bundle 70 comprises a camera and a microphone. The sensor bundle 70 may be phrased as a sensing device. The camera records vision data associated with the population of mixed-sex swine 90. Similarly, the microphone measures acoustic data associated with the population of mixed-sex swine 90. The camera and the microphone (i.e. the sensor bundle 70) measure occurrences of a behavioral feature of the population of mixed-sex swine 90. Here, the behavioral feature is biting of the at least one boar of the population of mixed-sex swine 90. Biting is a feature associated with the onset of puberty in boars, thus the sexual maturity of the at least one boar may be a function of the amount of biting. Moreover, biting is a behavioral feature that may be detected with the camera and subsequently counted, similarly, the microphone may measure increase in the amount of bites and reactions thereto by the population of mixed-sex swine 90.

The sensor bundle 70 subsequently provides (or forwards, or conveys) said vision data and said acoustic data to the controller 51 via a first signal. The first signal therefore comprises the vision data and the acoustic data, and the first signal is hence indicative of the behavioral feature (i.e. biting) that characterizes the sexual maturation level of the at least one boar within the population of mixed-sex swine. The controller 51 itself then determines the sexual maturation level of the at least one boar 90 based on the first signal by counting the number of occurrences of said feature (of biting). In alternative examples, this may be supported or aided by image analysis tools comprising artificial intelligence and/or machine learning algorithms, so as to improve an analysis of the vision data. Alternatively, the controller 51 may also assess the intensity of said occurrences, i.e. e.g. weighting each occurrence. Said sexual maturation level may for example be an average sexual maturation level.

Thus, the controller 51 determines the sexual maturation level of at least one boar within said population based on the first signal. The controller 51 subsequently determines whether the sexual maturity level of the at least one boar exceeds a predefined sexual maturity level. The predefined sexual maturity level is the onset of puberty of a boar. Upon determining that the sexual maturity level of the at least one boar exceeds the predefined sexual maturity level, the controller 51 controls the lighting device 60 to adapt said light recipe 61. Here, the length of the photo-period of said light recipe is increased, the light intensity of said light recipe is increased, and the blue content of the spectrum of the light recipe 61 is increased. This adaptation delays the sexual maturation of the at least one boar, because parameters of the light recipe 21 are adapted accordingly as discussed. Boar taint may therefore be reduced and/or prevented, because the population of boars will be less sexually mature upon slaughter. The light recipe 21 may alternatively also be optimized to both serving the population of mixed-sex swine, as well as to serve the function of delaying sexual maturation of the boars therein.

Alternatively, in examples not depicted: The behavioral feature may be any other behavioral feature characterizing the sexual maturation level of the at least one boar, or a combination of behavioral features. For example, an occurrence of the at least one boar fighting another animal, jumping onto another animal, being agitated, and/or performing a sexual activity. Similarly, mutatis mutandis, the feature may also be a physiological feature characterizing the sexual maturation level of the at least one boar, such as a musculature, a testicular size, a body smell, a skin status, a stress level, a perspiration rate, a hormone level, a pheromone level, and/or a core temperature. Thereby, in both alternatives, and sub-alternatives, a corresponding sensing device may be envisioned to determine (or detect, or analyze, or measure) one of said behavioral and/or physiological feature.

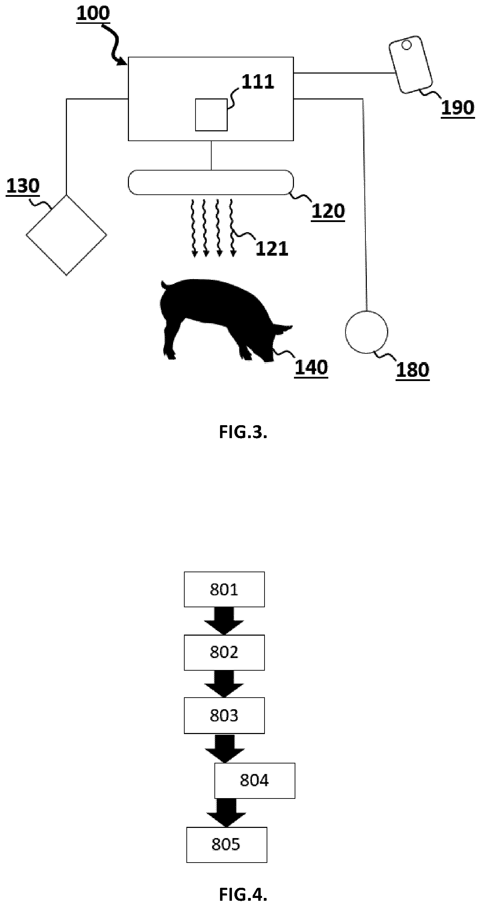

Figure 3 depicts schematically, by non-limiting example, a control system 100 according to the invention, which is similar to the embodiment depicted in figure 1, but may alternatively be similar to the embodiment depicted in figure 2. This embodiment however comprises a single boar. Moreover, the embodiment depicted in figure 3 comprises a control system 100 comprising a controller 111 receiving a second signal. The second signal is indicative of an average weight of the boar 140. Again, the control system 100 comprises a lighting device 120 emitting the light scene 121 and a processing device 130.

Here, the controller 111 receives the second signal from a device 180 for determining the weight of the boar 140. Such a device may for example be a camera or a Time-of-Flight sensor. The controller 111 determines the average weight of the boar 140 based on said second signal, and determines whether the average weight of the boar 140 exceeds a predefined average weight of the boar 140. The controller may for example determine said predefined average weight from a prestored growth curve of the boar. Upon determining that the average weight exceeds the predefined average weight, the controller 111 outputs a harvesting signal for harvesting the boar 140. This harvesting signal is a notification signal sent via a wireless connection to a user device 190, such as a user device 190 associated with the stable holder. Alternatively, said harvesting signal may be sent via a wireless connection, or conveyed via different media to an addressee or stable management system. Thus, the boar may advantageously be harvested when the boar reaches the stage of sexual maturity corresponding to the predefined sexual maturity level, i.e. e.g. before reaching puberty so as to reduce and/or prevent boar taint. Alternatively, the controller may control the lighting device to output the harvesting signal, the harvesting signal may thereby be a visual cue. Yet alternatively, the harvesting signal may be a control signal to control a lighting device to output a visual cue, such as illumination illuminating the location of said boar; or the harvesting signal may be an identification signal of the boar, such that an external device receiving said identification signal is informed to harvest said particular boar.

In an alternative embodiment (not depicted), an external device may determine that the average weight of the boar exceeds a predefined average weight, and subsequently convey this as a second signal to the control system 100 and/or the controller 111 thereof. The second signal is thereby indicative of the average weight of the boar exceeding a predefined average weight. The external device may for example be a scale. The processing to determine the average weight and whether said average weight exceeds a predefined average weight of the boar is therefore done external to the controller.

Figure 4 depicts schematically, by non-limiting example, an embodiment of a method 800 according to the invention. The method 800 adapts a light recipe in a growing cycle of at least one boar to delay sexual maturation of said at least one boar. The method comprises the step 801 of controlling a lighting device to illuminate the at least one boar with said light recipe. The light recipe is characterized by a spectrum, a photoperiod, and a light intensity. The method 800 further comprises the step 802 of obtaining a first signal indicative of a sexual maturation level of at least one boar. The method 800 further comprises the step 803 of determining the sexual maturation level of the at least one boar based on the first signal. In an optional sub step 804, the first signal is indicative of at least one feature characterizing the sexual maturation level of the at least one boar, and the method 800 comprises determining the sexual maturation level of the at least one boar by counting a number of occurrences of said at least one feature. Alternatively, the intensity of the at least one feature may be assessed. The method 800 comprises a further step 805 of controlling, upon determining that sexual maturity level of the at least one boar exceeds a predefined sexual maturity level, the lighting device to adapt said light recipe by (i) increasing a blue content of a spectrum of said light recipe, (ii) increasing a length of a photo-period of said light recipe, and/or (iii) increasing a light intensity of said light recipe. The method may be a feedback-loop optimizing the light recipe provided.

In an alternative embodiment, not depicted, the method may comprise a step wherein the first signal is indicative of the sexual maturation level of the at least one boar exceeding a predefined sexual maturation level. The sexual maturation level may for example be the onset of puberty of the at least one boar. In such an embodiment, the first signal already provides the determination of the sexual maturity level of the at least one boar exceeding a predefined sexual maturity level, hence obtaining said first signal may be equivalent to said controller determining this condition.

Additionally, in an alternative embodiment, the method may comprise: obtaining a second signal indicative of an average weight of the at least one boar; outputting, upon determining that the average weight of the at least one boar exceeds a predefined average weight of the at least one boar, a harvesting signal for harvesting the at least one boar.

## Claims

1. A control system for adapting a light recipe in a growing cycle of at least one boar to delay sexual maturation of said at least one boar, wherein the control system comprises a controller configured to:
- control a lighting device to illuminate the at least one boar with said light recipe;
- obtain a first signal indicative of a sexual maturation level of the at least one boar;
- control, upon determining that the sexual maturity level of the at least one boar exceeds a predefined sexual maturity level, the lighting device to adapt said light recipe by (i) increasing a blue content of a spectrum of said light recipe, (ii) increasing a length of a photo-period of said light recipe, and/or (iii) increasing a light intensity of said light recipe.

2. The control system according to claim 1, wherein the first signal is indicative of the sexual maturation level of the at least one boar exceeding the predefined sexual maturation level.

3. The control system according to claim 1, wherein the controller is configured to:
- determine the sexual maturation level of the at least one boar based on the first signal;
- determine that the sexual maturity level of the at least one boar exceeds a predefined sexual maturity level.

4. The control system according to claim 3, wherein the first signal is indicative of at least one feature characterizing the sexual maturation level of the at least one boar;
wherein the controller is configured to determine the sexual maturation level of the at least one boar by counting a number of occurrences of said at least one feature, and/or by assessing an intensity of said at least one feature.

5. The control system according to claim 4, wherein the control system comprises a sensing device for determining said at least one feature; and wherein the controller is configured to retrieve or obtain said first signal from the sensing device.

6. The control system according to claim 5, wherein the sensing device is a camera for obtaining vision data and/or a microphone for measuring acoustic data; wherein the first signal comprises respectively the vision data and/or the acoustic data.

7. The control system according to any one of the preceding claims 4-6, wherein the at least one feature comprises at least one behavioral feature and/or at least one physiological feature of the at least one boar.

8. The control system according to claim 6, wherein said at least one behavioral feature comprises an occurrence of the at least one boar: fighting another animal, jumping onto another animal, biting, being agitated, and/or performing a sexual activity.

9. The control system according to any one of the preceding claims, wherein the control system comprises the lighting device.

10. The control system according to any one of the preceding claims 1-9, wherein the controller is configured to:
- obtain a second signal indicative of an average weight of the at least one boar;
- output, upon determining a second condition in which the average weight of the at least one boar exceeds a predefined average weight of the at least one boar, a harvesting signal for harvesting the at least one boar.

11. The control system according to claim 10, wherein the second signal is indicative of the average weight of the at least one boar exceeding a predefined average weight of the at least one boar.

12. A method of adapting a light recipe in a growing cycle of at least one boar to delay sexual maturation of said at least one boar, wherein the method comprises:
- controlling a lighting device to illuminate the at least one boar with said light recipe;
- obtaining a first signal indicative of a sexual maturation level of the at least one boar;
- controlling, upon determining that sexual maturity level of the at least one boar exceeds a predefined sexual maturity level, the lighting device to adapt said light recipe by (i) increasing a blue content of a spectrum of said light recipe, (ii) increasing a length of a photo-period of said light recipe, and/or (iii) increasing a light intensity of said light recipe.

13. The method according to claim 12, wherein the first signal is indicative of at least one feature characterizing the sexual maturation level of the at least one boar, wherein the method comprises:
- determining the sexual maturation level of the at least one boar by counting a number of occurrences of said at least one feature, and/or by assessing an intensity of said at least one feature.

14. The method according to any one of the preceding claims 12-13, wherein the method comprises:
- obtaining a second signal indicative of an average weight of the at least one boar;
- outputting, upon determining that the average weight of the at least one boar exceeds a predefined average weight of the at least one boar, a harvesting signal for harvesting the at least one boar.

15. A computer program product for a computing device, the computer program product comprising computer program code to perform the method of any one of the preceding claims 12-14 when the computer program product is run on a processing unit of the computing device.

## Patentansprüche

1. Steuersystem zum Anpassen eines Lichtrezeptes in einem Wachstumszyklus mindestens eines Ebers, um eine Geschlechtsreifung des mindestens einen Ebers zu verzögern, wobei das Steuersystem eine Steuereinrichtung umfasst, die konfiguriert ist zum:
- Steuern einer Beleuchtungsvorrichtung, um den mindestens einen Eber mit dem Lichtrezept anzuleuchten;
- Erhalten eines ersten Signals, hinweisend auf ein Geschlechtsreifungsniveau des mindestens einen Ebers;
- Steuern, nach einem Bestimmen, dass das Geschlechtsreifeniveau des mindestens einen Ebers ein vordefiniertes Geschlechtsreifeniveau überschreitet, der Beleuchtungsvorrichtung, um das Lichtrezept anzupassen durch (i) Erhöhen eines Blauanteils eines Spektrums des Lichtrezeptes, (ii) Erhöhen einer Länge einer Fotoperiode des Lichtrezeptes und/oder (iii) Erhöhen einer Lichtintensität des Lichtrezeptes.

2. Steuersystem nach Anspruch 1, wobei das erste Signal darauf hinweisend ist, dass das Geschlechtsreifungsniveau des mindestens einen Ebers das vordefinierte Geschlechtsreifungsniveau überschreitet.

3. Steuersystem nach Anspruch 1, wobei die Steuereinrichtung konfiguriert ist zum:
- Bestimmen des Geschlechtsreifungsniveaus des mindestens einen Ebers basierend auf dem ersten Signal;
- Bestimmen, dass das Geschlechtsreifeniveau des mindestens einen Ebers ein vordefiniertes Geschlechtsreifeniveau überschreitet.

4. Steuersystem nach Anspruch 3, wobei das erste Signal auf mindestens ein Merkmal hinweisend ist, das das Geschlechtsreifungsniveaus des mindestens einen Ebers charakterisiert;
wobei die Steuereinrichtung konfiguriert ist, um das Geschlechtsreifungsniveau des mindestens einen Ebers durch Zählen einer Anzahl von Vorkommen des mindestens einen Merkmals und/oder durch Bewertung einer Intensität des mindestens einen Merkmals zu bestimmen.

5. Steuersystem nach Anspruch 4, wobei das Steuersystem eine Abtastvorrichtung zum Bestimmen des mindestens einen Merkmals umfasst; und wobei die Steuereinrichtung konfiguriert ist, um das erste Signal von der Abtastvorrichtung abzurufen oder zu erhalten.

6. Steuersystem nach Anspruch 5, wobei die Abtastvorrichtung eine Kamera zum Erhalten von Sichtdaten und/oder ein Mikrofon zum Messen von akustischen Daten ist; wobei das erste Signal jeweils die Sichtdaten und/oder die akustischen Daten umfasst.

7. Steuersystem nach einem der vorstehenden Ansprüche 4 bis 6, wobei das mindestens eine Merkmal mindestens ein Verhaltensmerkmal und/oder mindestens ein physiologisches Merkmal des mindestens einen Ebers umfasst.

8. Steuersystem nach Anspruch 6, wobei das mindestens eine Verhaltensmerkmal ein Vorkommen umfasst, dass der mindestens eine Eber: ein anderes Tier bekämpft, auf ein anderes Tier springt, beißt, erregt ist und/oder eine geschlechtliche Aktivität durchführt.

9. Steuersystem nach einem der vorstehenden Ansprüche, wobei das Steuersystem die Beleuchtungsvorrichtung umfasst.

10. Steuersystem nach einem der Ansprüche 1 bis 9, wobei die Steuereinrichtung konfiguriert ist zum:
- Erhalten eines zweiten Signals, das auf ein durchschnittliches Gewicht des mindestens einen Ebers hinweisend ist;
- Ausgeben, nach dem Bestimmen einer zweiten Bedingung, in der das durchschnittliche Gewicht des mindestens einen Ebers ein vordefiniertes durchschnittliches Gewicht des mindestens einen Ebers überschreitet, eines Schlachtsignals zum Schlachten des mindestens einen Ebers.

11. Steuersystem nach Anspruch 10, wobei das zweite Signal darauf hinweisend ist, dass das durchschnittliche Gewicht des mindestens einen Ebers ein vordefiniertes durchschnittliches Gewicht des mindestens einen Ebers überschreitet.

12. Verfahren zum Anpassen eines Lichtrezeptes in einem Wachstumszyklus mindestens eines Ebers, um eine Geschlechtsreifung des mindestens einen Ebers zu verzögern, wobei das Verfahren umfasst:
- Steuern einer Beleuchtungsvorrichtung, um den mindestens einen Eber mit dem Lichtrezept anzuleuchten;
- Erhalten eines ersten Signals, hinweisend auf ein Geschlechtsreifungsniveau des mindestens einen Ebers;
- Steuern, nach dem Bestimmen, dass das Geschlechtsreifeniveau des mindestens einen Ebers ein vordefiniertes Geschlechtsreifeniveau überschreitet, der Beleuchtungsvorrichtung, um das Lichtrezept anzupassen durch (i) Erhöhen eines Blauanteils eines Spektrums des Lichtrezeptes, (ii) Erhöhen einer Länge einer Fotoperiode des Lichtrezeptes und/oder (iii) Erhöhen einer Lichtintensität des Lichtrezeptes.

13. Verfahren nach Anspruch 12, wobei das erste Signal auf mindestens ein Merkmal hinweisend ist, das das Geschlechtsreifungsniveau des mindestens einen Ebers charakterisiert, wobei das Verfahren umfasst:
- Bestimmen des Geschlechtsreifungsniveaus des mindestens einen Ebers durch Zählen einer Anzahl von Vorkommen des mindestens einen Merkmals und/oder durch Bewertung einer Intensität des mindestens einen Merkmals.

14. Verfahren nach einem der vorstehenden Ansprüche 12 bis 13, wobei das Verfahren umfasst:
- Erhalten eines zweiten Signals, das auf ein durchschnittliches Gewicht des mindestens einen Ebers hinweisend ist;
- Ausgeben, nach dem Bestimmen, dass das durchschnittliche Gewicht des mindestens einen Ebers ein vordefiniertes durchschnittliches Gewicht des mindestens einen Ebers überschreitet, eines Schlachtsignals zum Schlachten des mindestens einen Ebers.

15. Computerprogrammprodukt für eine Rechenvorrichtung, das Computerprogrammprodukt umfassend Computerprogrammcode, um das Verfahren nach einem der vorstehenden Ansprüche 12 bis 14 durchzuführen, wenn das Computerprogrammprodukt auf einer Verarbeitungseinheit der Rechenvorrichtung ausgeführt wird.

## Revendications

1. Système de commande permettant d'adapter une recette de lumière dans un cycle de croissance d'au moins un verrat pour retarder la maturation sexuelle dudit au moins un verrat, le système de commande comprenant un organe de commande configuré pour :
- commander un dispositif d'éclairage pour illuminer l'au moins un verrat avec ladite recette de lumière ;
- obtenir un premier signal indiquant un niveau de maturation sexuelle de l'au moins un verrat ;
- commander, lorsqu'on détermine que le niveau de maturité sexuelle de l'au moins un verrat dépasse un niveau de maturité sexuelle prédéfini, le dispositif d'éclairage pour adapter ladite recette de lumière par (i) l'augmentation d'une teneur en bleu d'un spectre de ladite recette de lumière, (ii) l'augmentation d'une longueur d'une photopériode de ladite recette de lumière, et/ou (iii) l'augmentation d'une intensité de lumière de ladite recette de lumière.

2. Système de commande selon la revendication 1, dans lequel le premier signal indique le niveau de maturation sexuelle de l'au moins un verrat dépassant le niveau de maturation sexuelle prédéfini.

3. Système de commande selon la revendication 1, dans lequel le dispositif de commande est configuré pour :
- déterminer le niveau de maturation sexuelle de l'au moins un verrat en fonction du premier signal ;
- déterminer que le niveau de maturité sexuelle de l'au moins un verrat dépasse un niveau de maturité sexuelle prédéfini.

4. Système de commande selon la revendication 3, dans lequel le premier signal indique au moins une caractéristique caractérisant le niveau de maturation sexuelle de l'au moins un verrat ;
dans lequel l'organe de commande est configuré pour déterminer le niveau de maturation sexuelle de l'au moins un verrat en comptant un nombre d'occurrences de ladite au moins une caractéristique, et/ou en évaluant une intensité de ladite au moins une caractéristique.

5. Système de commande selon la revendication 4, dans lequel le système de commande comprend un dispositif capteur permettant de déterminer ladite au moins une caractéristique ; et dans lequel l'organe de commande est configuré pour récupérer ou obtenir ledit premier signal à partir du dispositif capteur.

6. Système de commande selon la revendication 5, dans lequel le dispositif capteur est une caméra permettant d'obtenir des données de vision et/ou un microphone permettant de mesurer des données acoustiques ; dans lequel le premier signal comprend respectivement les données de vision et/ou les données acoustiques.

7. Système de commande selon l'une quelconque des revendications précédentes 4 à 6, dans lequel l'au moins une caractéristique comprend au moins une caractéristique comportementale et/ou au moins une caractéristique physiologique de l'au moins un verrat.

8. Système de commande selon la revendication 6, dans lequel ladite au moins une caractéristique comportementale comprend une occurrence de l'au moins un verrat : combattant un autre animal, sautant sur un autre animal, mordant, étant agité, et/ou mettant en oeuvre une activité sexuelle.

9. Système de commande selon l'une quelconque des revendications précédentes, le système de commande comprenant le dispositif d'éclairage.

10. Système de commande selon l'une quelconque des revendications 1 à 9 précédentes, dans lequel l'organe de commande est configuré pour :
- obtenir un second signal indiquant un poids moyen de l'au moins un verrat ;
- délivrer en sortie, lorsqu'on détermine qu'une seconde condition dans laquelle le poids moyen de l'au moins un verrat dépasse un poids moyen prédéfini de l'au moins un verrat, un signal de collecte pour collecter l'au moins un verrat.

11. Système de commande selon la revendication 10, dans lequel le second signal indique le poids moyen de l'au moins un verrat dépassant un poids moyen prédéfini de l'au moins un verrat.

12. Procédé d'adaptation d'une recette de lumière dans un cycle de croissance d'au moins un verrat pour retarder la maturation sexuelle dudit au moins un verrat, le procédé comprenant :
- la commande d'un dispositif d'éclairage pour illuminer l'au moins un verrat avec ladite recette de lumière ;
- l'obtention d'un premier signal indiquant un niveau de maturation sexuelle de l'au moins un verrat ;
- la commande, lorsqu'on détermine que le niveau de maturité sexuelle de l'au moins un verrat dépasse un niveau de maturité sexuelle prédéfini, du dispositif d'éclairage pour adapter ladite recette de lumière par (i) l'augmentation d'une teneur en bleu d'un spectre de ladite recette de lumière, (ii) l'augmentation d'une longueur d'une photopériode de ladite recette de lumière, et/ou (iii) l'augmentation d'une intensité de lumière de ladite recette de lumière.

13. Procédé selon la revendication 12, dans lequel le premier signal indique au moins une caractéristique caractérisant le niveau de maturation sexuelle de l'au moins un verrat, le procédé comprenant :
- la détermination du niveau de maturation sexuelle de l'au moins un verrat en comptant un nombre d'occurrences de ladite au moins une caractéristique, et/ou en évaluant une intensité de ladite au moins une caractéristique.

14. Procédé selon l'une quelconque des revendications 12 à 13 précédentes, le procédé comprenant :
- l'obtention d'un second signal indiquant un poids moyen de l'au moins un verrat ;
- la sortie, lorsqu'on détermine que le poids moyen de l'au moins un verrat dépasse un poids moyen prédéfini de l'au moins un verrat, d'un signal de collecte pour collecter l'au moins un verrat.

15. Produit programme informatique pour un dispositif informatique, le produit programme informatique comprenant un code de programme informatique pour mettre en oeuvre le procédé selon l'une quelconque des revendications 12 à 14 précédentes lorsque le produit programme informatique est exécuté sur une unité de traitement du dispositif informatique.
